## Europäisches Patentamt

(19) European Patent Office

Office européen des brevets

(11) Publication number: **0 066 284**
**B1**

## EUROPEAN PATENT SPECIFICATION

(45) Date of publication of patent specification: **30.12.86**

(51) Int. Cl.⁴: **C 12 N 5/00**

(21) Application number: **82104738.8**

(22) Date of filing: **28.05.82**

(54) **Culture medium and its use.**

(30) Priority: **28.05.81 JP 81600/81**

(43) Date of publication of application:
**08.12.82 Bulletin 82/49**

(45) Publication of the grant of the patent:
**30.12.86 Bulletin 86/52**

(84) Designated Contracting States:
**CH DE FR GB IT LI NL SE**

(56) References cited:
**EP-A-0 017 570**

**CHEMICAL ABSTRACTS, vol. 96, no. 13, 29th March 1982, page 375, no. 100488r, Columbus, Ohio, USA I. YAMANE et al.: "Alpha-Cyclodextrin, a novel substitute for bovine albumin in serum-free culture of mammalian cells"**

**CHEMICAL ABSTRACTS, vol. 77, no. 1, 3rd July 1972, page 244, no. 2537u, Columbus, Ohio, USA**

(73) Proprietor: **AJINOMOTO CO., INC.**
**5-8, Kyobashi 1-chome, Chuo-ku**
**Tokyo 104 (JP)**

(73) Proprietor: **Yamane, Isao**
**No. 6-6-20, Minami Yagiyama**
**Sendai-shi Miyagi-ken (JP)**

(72) Inventor: **Yamane, Isao**
**No. 6-6-20, Minami Yagiyama**
**Sendai-shi Miyagi-ken (JP)**
Inventor: **Kan, Mikio**
**No. 506, Kodan Apat 19-to No. 2-5, Tsurugaya**
**Sendai-shi Miyagi-ken (JP)**
Inventor: **Minamoto, Yoshiki**
**No. 5, Zenbu-cho Asaki-ku**
**Yokohama-shi Kanagawa-ken (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

Courier Press, Leamington Spa, England.

## Description

This invention relates to some improvements in culture media, according to which a substitute composition for serum for culture media may be provided, and hence the serum concentration in a culture medium for mammalian cells may be reduced, or the serum usually contained in a culture medium may be replaced completely.

So far, culture media for the proliferation of mammalian cells are generally prepared by adding a large amount of serum and, therefore, conventional culture media usually contain a large amount of serum. In connection with such culture media, the following problems arise, for example:

(a) For the production of interferons by human cells, or for the propagation of virus in order to prepare vaccine, for example, fetal bovine serum or calf serum is usually added in amounts of around 10% to these culture media. Therefore, more than half of the cost of such media is usually occupied by such sera, and these media will be expensive.

(b) In addition, there is the troublesome necessity of checking up the serum lots in advance before the sera are used to prepare culture media, because these sera might be contaminated with mycoplasma or virus, or the quality of these sera often differs from lot to lot.

(c) Further, these sera contain various and unidentified proteins derived from cattle or horse, which make it difficult to isolate products such as interferon from the remainder of the culture medium.

There has been known a medium for the clonal growth of mammalian cells, which contains linoleic acid in glucose together with other powdery ingredients (C.A. 77, No. 1, 1982, p. 244, No. 2537 u).

The inventors had attempted to decrease the serum concentration in a culture medium or to develop serum-free culture media, and found that serum albumin may act as a substitute for sera and that mammalian cells grow in a culture medium containing serum albumin without any other serum components as well as in conventional serum-containing culture media. The inventors have filed a patent application for an invention based on these findings (Japanese patent application No. 149,731/1980).

The inventors further tried to develop another medium having decreased serum concentration or even serum-free culture medium, which is easier to prepare or handle and accordingly is less expensive.

This objective is attained according to the invention by a culture medium for mammalian cell cultures, comprising known nutrients, additives and serum or serum albumin, which is characterized in that the serum or serum albumin component is wholly or partially replaced by cyclodextrin and at least one lipophilic substance as a nutrient selected from unsaturated or saturated fatty acids and their glycerides, and lipophilic vitamins.

The present invention further is directed to the use of a mixture of or of an inclusion complex between cyclodextrin and at least one lipophilic substance useful as a cell culture nutrient as a substitute for serum or serum albumin in a culture medium for mammalian cells.

This new serum-free culture medium has been found to allow the growth of mammalian cells in the presence of less or even without using any serum albumin or serum as usually employed in a conventional serum-containing medium, or a known serum-free culture medium.

It has been very difficult to add water-insoluble or sparingly soluble unsaturated or saturated fatty acids and other water-insoluble or sparingly soluble nutrients to culture media without causing a cytotoxic effect.

The inventors were first successful in introducing these lipophilic nutrients into a serum-free culture medium by using a cyclodextrin as vehicle. This invention was established on basis of the finding that a cyclodextrin exerts no or a lower cytotoxicity against the growth of mammalian cells and is also an appropriate additive for introducing various lipophilic substances such as saturated or unsaturated fatty acids and lipophilic vitamins. In other words, the use of a cyclodextrin makes it easy to add lipophilic substances as nutrients to a serum-free culture medium.

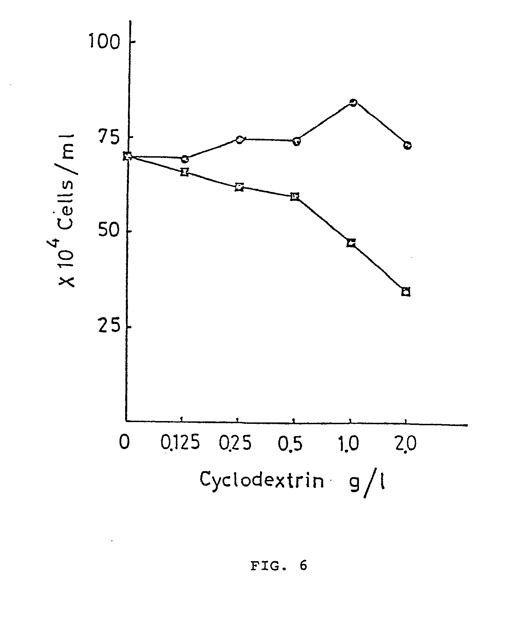

There are three cyclodextrins, i.e. α-, β- and γ-cyclodextrin, and all these cyclodextrins are employable for the present invention. Among them, α-cyclodextrin is the best for the purpose of this invention because of its lack of cytotoxicity against the growth of mammalian cells. β-Cyclodextrin is generally employed in a drug preparation, shows however some cytotoxic effect on the growth of mammalian cells. Therefore, β-cyclodextrin is not as good as α-cyclodextrin for that purpose at higher concentrations. (See Figure 6).

In this invention, cyclodextrin is used for introducing lipophilic substances as ingredients to be added as nutrients in a culture medium or for decreasing the cytotoxicity of such ingredients against mammalian cells.

The lipophilic substances include unsaturated fatty acids such as linoleic acid, linolenic acid, oleic acid, arachidonic acid and erucic acid and their glycerides, saturated fatty acids such as palmitic acid and stearic acid and their glycerides, and lipophilic vitamins such as vitamins A, D and E.

Cyclodextrin and the lipophilic substance may be added as they are (i.e., as such), or a mixture of cyclodextrin and a lipophilic substance may be added, but cyclodextrin and the lipophilic substance are preferably added to a culture medium after having been reacted with each other.

The reaction is, for example, carried out, as follows:

An aqueous solution of cyclodextrin and a solution of the lipophilic substance in a hydrophilic organic solvent such as an alcohol or dioxane are first prepared, then both solutions are mixed and, for example, heated up to 75°C or heated to 70°C and kept at this temperature for 5 minutes. The reaction product is, for example, precipitated by cooling the reaction mixture and it is easily separated in such precipitate form from the reaction mixture. The reaction product thus obtained is introduced into a culture medium.

The reaction product is usually in the form of an inclusion complex in which cyclodextrin includes the lipophilic substance inside, but it may be in the form in which cyclodextrin is bound to the lipophilic substance in some other way.

The amount of cyclodextrin to be used depends on the purposes of its use. When it is used in the form of its inclusion complex with a lipophilic substance, an inclusion complex is preferably used which results from reacting cyclodextrin with a lipophilic substance in a cyclodextrin/lipophilic substance molar ratio of 3 to 500:1 and usually 10 to 100:1.

For the preparation of a culture medium for mammalian cells, the addition of serum has been thought to be essential. A serum-free culture medium according to Japanese pat. appln. No. 149,731/1980 does not require serum as such, but still requires serum albumin, one component of serum.

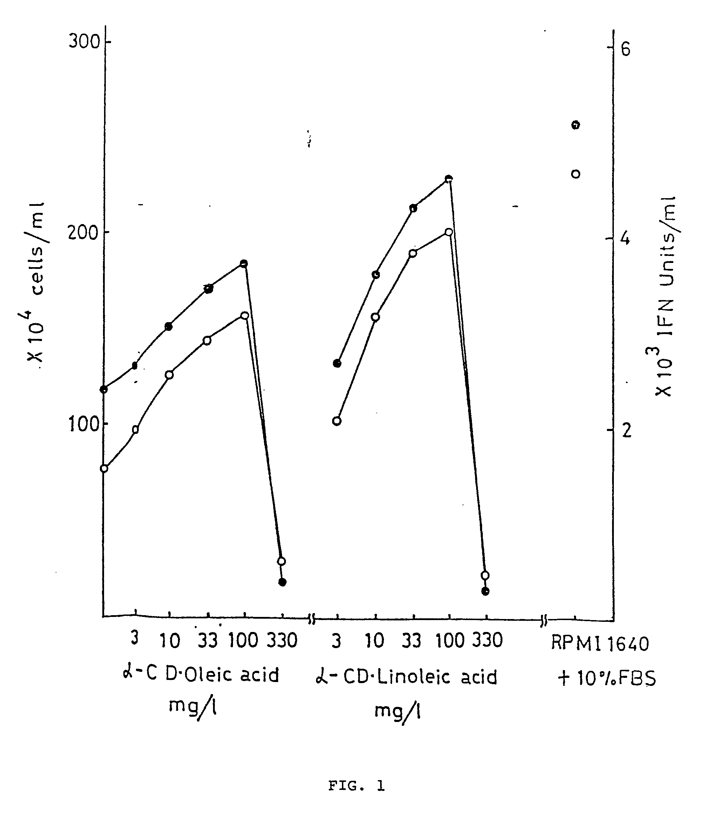

However, according to this invention, for example, in a serum-free culture medium which contains a reaction product of linoleic acid with α-cyclodextrin, instead of serum or serum albumin, mammalian cells can grow as well as in a conventional serum-containing medium and can produce physiologically active substances such as interferon. (See Figure 1).

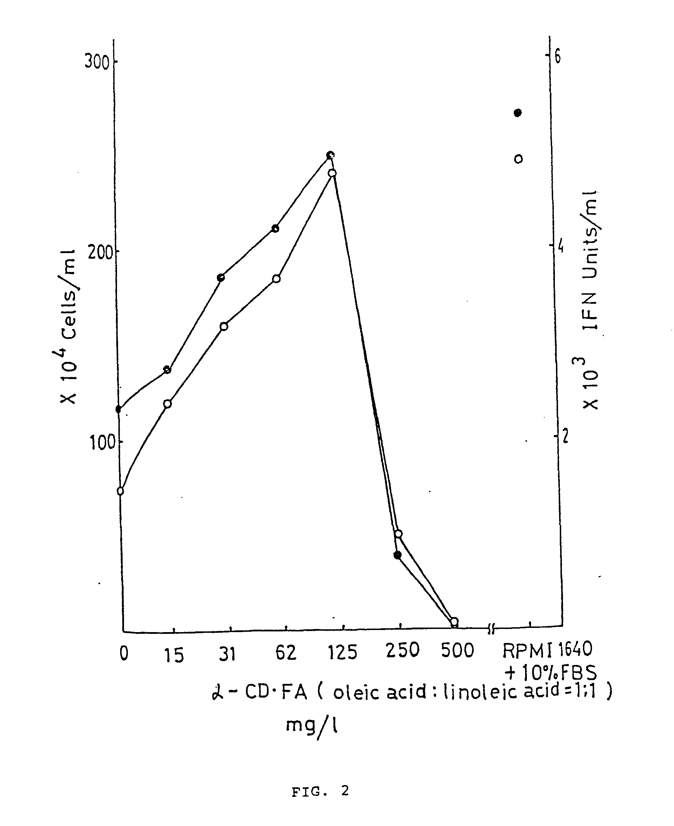

The cell growth and the production of physiologically active substances can further be improved when other unsaturated fatty acids such as oleic acid and linolenic acid, and/or vitamin E are added as their respective inclusion complexes with cyclodextrin, together with the linoleic acid/cyclodextrin inclusion complex. (See Figures 2, 3 and 5).

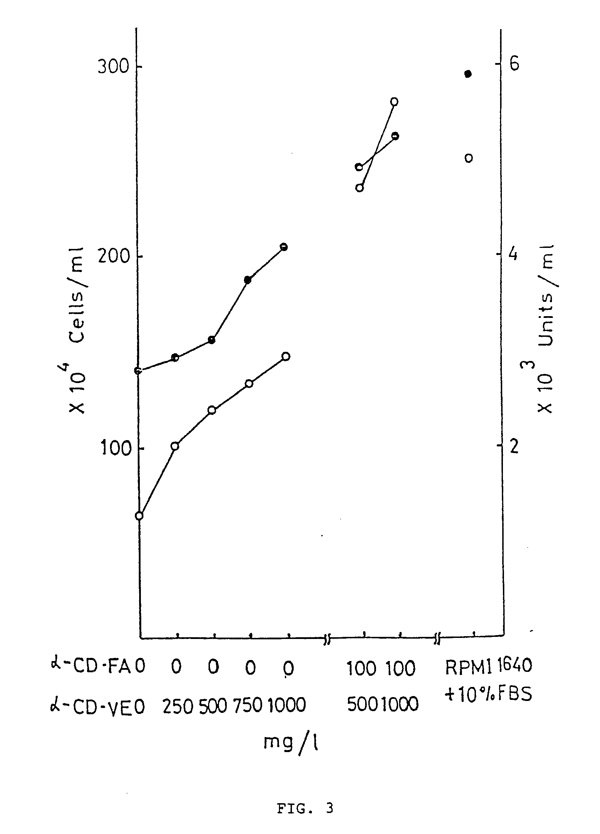

An inclusion complex prepared from vitamin E and cyclodextrin, even when singly used, can also improve the cell growth or enhance the productivity of interferon (See Figure 3). The same applies for an inclusion complex of cyclodextrin with one of the other unsaturated fatty acids such as oleic acid (See Figure 1).

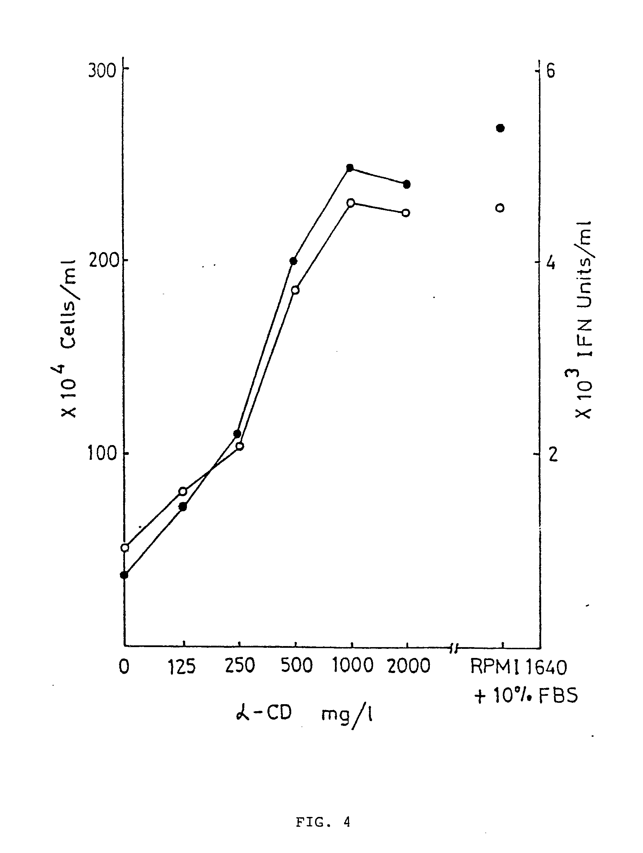

When an inclusion complex prepared from linoleic acid and α-cyclodextrin under the above-mentioned conditions is used as the substitute for serum or serum albumin, it is introduced in amounts of about 0.1 to 5 mg per liter of culture medium, preferably 0.5 to 2 mg per liter of culture medium, expressed in terms of the linoleic acid moiety. Usually, larger amounts of the linoleic acid moiety than the above will have an adverse effect on the cell growth, i.e., cytotoxicity. The lower limiting concentration of the linoleic acid moiety which will have the cytotoxic effects on cell growth will, however, be upgraded up to 10 mg per liter of culture medium, if α-cyclodextrin is additionally added into the medium in amounts of 300 to 1000 mg per liter of the culture medium (see Figure 4).

An inclusion complex of α-cyclodextrin with other fatty acids than linoleic acid, such as oleic acid, linolenic acid, palmitic acid and stearic acid, is preferably introduced in amounts of around 0.1 to 1 mg/l expressed in terms of the fatty acid moiety.

In case the inclusion complex prepared from vitamin E and α-cyclodextrin is added to the remainder of a serum-free or serum albumin-free culture medium, its optimal concentration is around 0.5 to 10 mg/l, preferably 1 to 5 mg/l, as expressed in terms of the concentration of vitamin E.

A serum- or serum albumin-free culture medium according to this invention contains as other ingredients cell growth factors such as insulin and human transferrin, and nucleic acid precursors such as hypoxanthine, thymidine, deoxyadenosine, and deoxycytidine, together with such carbon sources as glucose, amino acids, vitamins, minerals and other nutrients which are contained in a conventional serum-supplement medium or a known serum-free medium for mammalian cell cultures.

A cell growth factor is preferably used in a concentration of around 1 to 100 mg per liter of culture medium, and a nucleic acid precursor is preferably used in a concentration of around 0.01 to 50 mg per liter of culture medium. Two or more cell growth factors are used simultaneously or in combination, depending on the kind of mammalian cells. So are two or more nucleic acid precursors.

Glucose is usually employed as the carbon source in amounts of 0.5 to 10 g per liter of culture medium. Other carbon sources such as pyruvate are also adequately added.

Amino acids such as L-alanine, L-arginine, L-glutamine, L-methionine, L-threonine, L-lysine, L-valine and L-phenylalanine are also used, such amino acids being the components of proteins. Into a serum-containing culture medium, essential amino acids are mainly introduced, but various non-essential amino acids as well as essential amino acids are preferably introduced into a serum-free culture medium according to this invention. Amino acids are preferably added in total amounts of around 0.5 to 5 g per liter of culture medium. Other amino acids are preferably supplemented, in consideration of the usual serum-containing medium.

Vitamins such as ascorbic acid, riboflavin, thiamine · HCL, Ca-pantothenate, nicotic acid amide, pyridoxal · HCl, i-inositol, folic acid, vitamin $B_{12}$, and biotin are added, together with minerals such as NaCl, KCl, $CaCl_2$, $MgSO_4$, $NaH_2PO_4$, $FeSO_4$, $ZnSO_4$, and $NaSeO_3$, in adequate amounts.

In addition, such metabolic intermediates as choline-bitartrate, glutathione and putrescine · 2HCl, and buffering substances such as $NaHCO_3$, β-glycerophosphate · 2Na, and/or N-2-hydroxyethylpiperazine-N-2-ethane sulfonate are adequately supplemented.

In addition to the above ingredients and additives, the following basal media are referred to, in determining the composition of a culture medium according to this invention; Dulbecco's Modified Eagle Medium, RPMI-1640 Medium, Eagle Minimum Essential Medium and Ham F-12 Medium.

The method of preparing a culture medium according to this invention is not critical. It may be prepared, for example, by dissolving all the ingredients and additives in water in their respective appropriate concentrations first and then filtering the solution through a membrane filter under pressure to obtain a sterilized culture medium. It would be apparent from the above disclosure that cyclodextrin and a lipophilic substance are preferably used in the form of an inclusion complex between them.

Mammalian cells which can grow in a serum-free culture medium of this invention are not limited to specified cells, but a wide variety of cells such as lymphocytes, fibroblastes, epithelial cells, and their transformed cells, various neoplastic cells, and hybridomas derived therefrom can grow therein.

Examples of such mammalian cells include Epstein-Barr Virus (EBV)-transformed human lymphoblastoid cell lines such as UMCL and C5180Y, human Burkitt's lymphoma-derived Namalwa cells, murine lymphoid cell-derived myeloma SPI cells, human fibroblast cells such as HEL and LMR-90, human tumor-derived epithelial cells such as HeLa-S$_3$, Hep-2 and KB, human primary cultured cells, rat Yoshida sarcoma cells, hamster fibroblast cells BHK-21, murine fibroblast cells 3T3, and murine lymphoma cells YAC-1.

The method of culturing mammalian cells with the use of a culture medium of this invention is not critical, either.

Mammalian cells are cultured in a serum-free culture medium of this invention under the same or almost the same conditions as those for a conventional serum-containing medium; for example, cells are cultured in a culture medium at an initial cell density of $10^4$ to $10^6$ cells/ml and at 35 to 37°C under supplying 4 to 6% (v/v) $CO_2$-containing sterilized air into the culture vessel. Fibroblast cells are preferably cultured under lower $O_2$ supply (5 to 10% v/v) than atmosphere, especially in a serum-free medium.

The serum-free medium of this invention is employable not only for the cell growth of mammalian cells but also for the production of interferons, lymphokines, and antibodies by cultivating mammalian cells.

Serum-free media of this invention have been established on the basis of the inventor's findings that cyclodextrin does not show any cytotoxic effect on the cell growth and that lipophilic substances included in cyclodextrin still show such effects as cell growth promoting effect and accelerating effect of the productivity of valuable products. This invention has also been established on the finding that the solubility and stability in water of the culture medium ingredients are improved by including them in cyclodextrin as a vehicle.

In accordance with this invention, those substances which are water-insoluble, unstable in culture medium or cytotoxic as such and which accordingly were considered as being unemployable as culture medium ingredients have been made available. In other words, this invention will enlarge the availability of various ingredients and the ranges of researches on the development of new culture media.

It would be apparent from the foregoing disclosure, especially in connection with the effects of cyclodextrin on cell growth and/or some culture medium ingredients, that a conventional serum culture medium may be made more effective when it is added with cyclodextrin because some lipophilic substances originating from serum are contained in such a conventional medium. A culture medium containing serum albumin at its lower concentrations instead of serum may also be made more effective when added with cyclodextrin because commercial serum albumin products such as crystalline albumin and Cohn's Fraction V are usually accompanied by lipophilic substances. An animal cell culture medium comprising cyclodextrin as well as ordinary nutrients and additives including serum or serum albumin is accordingly in accordance with this invention.

It would also be very apparent that the serum moiety or the serum albumin moiety in a conventional serum culture medium may be partially replaced by at least one lipophilic substance nutrient and cyclodextrin, said lipophilic substance and cyclodextrin being preferably in the form of an inclusion complex between them. A conventional animal cell culture medium in which the serum moiety or the serum albumin moiety is partially replaced by at least one lipophilic substance nutrient and cyclodextrin is therefore also in accordance with this invention.

A nutrient substitute composition for serum for an animal cell culture comprising at least one lipophilic substance nutrient and cyclodextrin may be put on the market as such and it is also in accordance with this invention.

If one takes a lipid as ingredient, for example linoleic acid, linoleic acid shows almost no growth promoting effects at a concentration lower than 1 mg/l, when linoleic acid itself is directly added in a culture medium, while this fatty acid at a concentration of more than 1 mg/l shows a cytotoxic effect on the cell growth. However, if it is added in the form of its inclusion complex with α-cyclodextrin to the culture medium, the upper limit of the fatty acid concentration which shows cytotoxicity is raised to 10 mg per liter of culture medium and at a lower concentration than this concentration, mammalian cells grow well and show sufficient productivity of physiologically active substances, such as interferon.

Based on these new findings, the inventors have been successful in dispensing with, or reducing the amount of serum which had been essential for the cell growth but was very expensive and appeared to be difficult to obtain, and established a new culture medium which contains no or less proteinaceous substances. At the same time the inventors did open the way to sterilize the culture medium by autoclave heating. Use of a culture medium of this invention facilitates the isolation and purification of the product accumulated in the culture medium. Also the mass production of culture medium is made easier according to this invention.

It is necessary to add serum albumin or an expensive cell-attachment factor such as fibronectin to the medium when fibroblast cells are cultivated in an ordinary serum albumin-containing medium. There is however no need to add fibronectin if an inclusion complex prepared from α-cyclodextrin and an unsaturated fatty acid such as linoleic acid is added to the medium.

Further, there have not been known any good means of introducing lipophilic vitamins such as vitamin E into a culture medium without an organic solvent, though such vitamins have been considered as essential for cell growth, according to this invention however these nutrients may be added in satisfactory amounts to the culture medium.

This invention will be explained in greater detail with reference to the following examples.

The interferon activity produced was determined in terms of anti-viral activity using FL cells and Vesicular Stomatitis Virus in the following examples.

Example 1 (Propagation of UMCL cells)

Fresh heparinized umbilical cord blood, 20 ml, was obtained from a new-born infant. Then the cord blood lymphocytes were quickly isolated by the Ficoll isopaque density gradient method. The cord blood lymphocyte fraction was mixed with 3 times the volume of Eagle's Minimum Essential Medium (Nissui Pharmaceutical Co.) and the mixture was centrifuged. The supernatant was discarded. After repeating this treatment 3 times, the washed lymphocytes were suspended at a cell density of $3\times10^6$/ml in Culture Medium RPMI-1640 (Nissui Pharmaceutical Co.) containing 10 v/v% fetal bovine serum.

To this lymphocyte suspension, EBV (Epstein Barr Virus) grown in B-95-8 cells was added in a concentration of $5\times10^5$ $TD_{50}$/ml, and this mixture was incubated at 37°C for 2 hours, followed by harvesting the lymphocytes with a centrifuge. After washing these lymphocytes with Eagle's MEM medium 3 times, the lymphocytes were resuspended at a cell density of 3 to $10^6$/ml in Culture Medium RPMI-1640 containing 10 v/v% fetal bovine serum and cultivated at 36 to 37°C for 1.5 months in a humidified atmosphere containing 5% $CO_2$ in air. During this cultivation, fresh RPMI-1640 medium containing 10 v/v% fetal bovine serum was added in equal volume or half volume of the culture medium and the medium was exchanged with this fresh medium every 5 days.

Two additional samples of umbilical cord blood from new-born infants were processed in the same way and 3 samples in total of lymphoblastoid cells, i.e. UMCL-1, UMCL-2 and UMCL-3, were prepared.

The spontaneous interferon production of these transformed cells was assayed and the results are shown in Table 1.

The assay was carried out as follows: Each lymphoblastoid cell line was cultivated at an initial cell density of $5\times10^5$/ml in Culture Medium RPMI-1640 containing 10 v/v% fetal bovine serum at 37°C for 5 days in a humidified atmosphere containing 5% $CO_2$ in air and the interferon activity in the supernatant was measured.

TABLE 1

| Cell line | Interferon activity |
|-----------|---------------------|
| UMCL-1 | 2.5 ($10^3$ U/ml) |
| UMCL-2 | 6.1 |
| UMCL-3 | 4.5 |

In connection with this example, reference is made to T. Sato et al., Exp. Cell Res., *138*, 127 (1982).

Example 2

A culture medium named RITC 56-1 was prepared by dissolving in a Dulbecco's Modified Eagle Medium having the composition shown under A the additional nutrients and additives in the given amounts shown under B.

A (Dulbecco's Modified Eagle Medium):

| | |
|---|---|
| NaCl | 6400.0 (mg/l) |
| KCl | 400.0 |
| CaCl$_2$ (non-hydrate) | 200.0 |
| MgSO$_4$ (non-hydrate) | 97.7 |
| NaH$_2$PO$_4$ · 2H$_2$O | 125.0 |
| Fe(NO$_3$)$_3$ · 9H$_2$O | 0.1 |
| Glucose | 1000.0 |
| Na pyruvate | 110.0 |
| L-arginine · HCl | 84.0 |
| L-Cystine · 2HCl | 62.6 |
| glycin | 30.0 |
| L-glutamine | 584.0 |
| L-histidine · HCl · H$_2$O | 42.0 |
| L-isoleucine | 104.8 (mg/l) |
| L-leucine | 104.8 |
| L-lysine · HCl | 146.2 |
| L-Methionine | 30.0 |
| L-phenylalanine | 66.0 |
| L-serine | 42.0 |
| L-threonine | 95.2 |
| L-tryptophan | 16.0 |
| L-tyrosinate · 2Na | 89.5 |
| L-valine | 93.6 |
| choline bitartrate | 7.2 |
| folic acid | 4.0 |
| nicotinic acid amide | 4.0 |
| Ca pantothenate | 4.0 |
| pyridoxal · HCl | 4.0 |
| riboflavin | 0.4 |
| thiamine · HCl | 4.0 |
| i-inositol | 7.2 |
| Phenol red | 5.0 |

B (Additional nutrients):

| | |
|---|---|
| insulin | 10 (mg/l) |
| human transferrin | 5 |
| hypoxanthine | 4 |
| thymidine | 0.7 |
| deoxycytidine | 0.03 |
| deoxyadenosine | 1.0 (mg/l) |
| 6.8-dihydroxypurine | 0.3 |
| glucose | 1000 |
| mannose | 500 |
| galactose | 500 |
| lecithin | 2.5 |
| cholesterol | 1 |
| L-alanine | 20 |
| L-asparagine · H$_2$O | 56 |
| L-aspartic acid | 20 |
| L-cysteine · HCl · H$_2$O | 40 |
| L-glutamic acid | 20 |
| L-proline | 20 |
| ascorbic acid | 10 |
| biotin | 0.2 |
| folinic acid | 0.01 |
| vitamin B$_{12}$ | 0.1 |
| FeSO$_4$ · 7H$_2$O | 0.8 |
| ZnSO$_4$ · 7H$_2$O | 0.02 |
| Na$_2$SeO$_3$ | 0.004 |
| CaCl$_2$ | 100 |

| | |
|---|---|
| glutathione | 1.0 |
| putrescine · 2HCl | 0.1 |
| β-glycerophosphate · 2Na | 1500 |
| NaHCO$_3$ | 1300 |

On the other hand, the inclusion complex between α-cyclodextrin (α-CD) and linoleic acid, oleic acid or vitamin E (VE) was prepared as follows:

1 g of α-CD was dissolved in 7 ml of distilled water, and 10 mg of linoleic acid, oleic acid or vitamin E was dissolved in 7 ml of ethanol. Both the aqueous and ethanolic solutions were mixed and the mixture was heated to, and kept at 70°C under bubbling with nitrogen gas until the mixture became opalescent. The opalescent solution was immediately left at room temperature, and then kept at 4°C for additional 20 hrs after it had been cooled to the room temperature. The precipitate formed, i.e., inclusion complex, was collected by centrifuging, slightly washed once with 10 ml of ethanol and dried in vaccuo. The resulting powder was washed two times with petroleum-ether and dried in vaccuo.

Figures 1 to 4 show the cell density and interferon production in the culture medium when an UMCL-3 cell line was cultivated in the RITC 56-1 culture medium supplemented with one or more inclusion complexes (Figures 1 to 3) and further with α-cyclodextrin (Figure 4).

The procedures are as follows:

RITC 56-1 culture medium was supplemented with one or more inclusion complexes and further α-cyclodextrin, as the case may be, as shown in Figures 1 to 4 in the respective amounts stated in the same figures. The resulting solution was sterilized by filtering through a membrane filter. In the resulting medium UMCL-3 cells were inoculated at an initial cell density of $5 \times 10^5$ cells/ml, and cultivated for 5 days at 37°C under 5% CO$_2$-95% air. The cell density and interferon production in the culture medium was measured.

In connection with Figure 4, the culture media were RITC 56-1 culture media supplemented with 300 mg/l of the inclusion complex of α-cyclodextrin with linoleic acid and further with α-cyclodextrin in the amounts given in the figure.

The same cell line was cultivated in RPMI-1640 culture medium supplemented with 10 v/v% fetal bovine serum, and the results are shown on the right side in all of the figures for the purpose of comparison.

In all of the figures, the closed circles and open circles indicate the concentrations of viable cells and interferon production, respectively.

Example 3

A culture medium named RITC 80-7 was prepared by using an MEM medium and some other nutrients and additives.

The composition of the RITC 80-7 medium is as follows:

| MEM medium (Nissui | |
|---|---|
| Pharmaceutical Co.) | 9400 (mg/l) |
| L-aspartic acid | 13.3 |
| L-glutamine | 292 |
| glycine | 7.5 |
| L-glutamic acid | 0.15 (mg/l) |
| L-proline | 3.5 |
| L-serine | 10.5 |
| folinic acid | 0.00005 |
| 3,3',5-triiodo-L-thyronine | 0.0002 |
| mouse-EGF | 0.01 |
| human transferrin | 10 |
| insulin | 1 |
| vitamin $B_{12}$ | 0.02 |
| biotin | 0.02 |
| putrescine · 2HCl | 0.02 |
| Na pyruvate | 110 |
| Choline chloride | 16 |
| thymidine | 0.07 |
| hypoxanthine | 0.24 |
| $CuSO_4 · 5H_2O$ | 0.0000025 |
| $FeSO_4 · 7H_2O$ | 0.8 |
| $MnSO_4 · 7H_2O$ | 0.0000024 |
| $(NH_4)_6Mo_7O_{24} · H_2O$ | 0.0012 |
| $NiCl_2 · 6H_2O$ | 0.000012 |
| $NH_4VO_3$ | 0.000058 |
| $H_2SeO_3$ | 0.00039 |
| N-2-hydroxyethylpiper- | |
| azine-N-2-ethane | |
| sulfonic acid | 3300 |
| NaOH | 300 |
| $NaHCO_3$ | 1400 (mg/l) |

In connection with the above RITC 80-7 culture medium, reference is made to I. Yamane et al., Exp. Cell Res., *134*, 470 (1981).

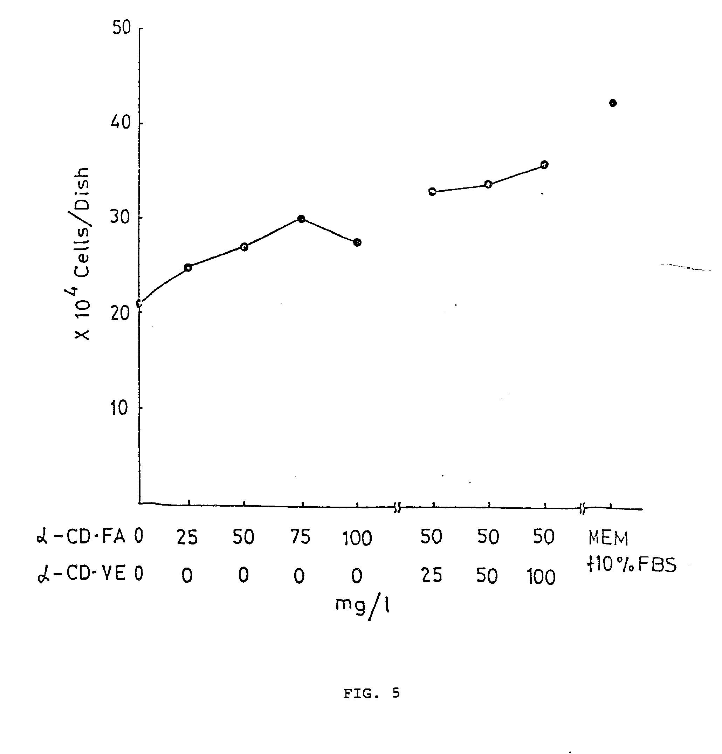

The inclusion complexes given in Figure 5 of α-cyclodextrin and unsaturated fatty acids or vitamin E prepared by the same method as in Example 2 was added in the amounts given in the same figure to RITC 80-7 medium, and the resulting culture medium solution was sterilized by filtration on a membrane filter.

1.5 ml of the sterile medium was pipetted into each culture dish with 3.5 cm diameter (Lux Co.). Human embryonic lung diploid fibroblast cells cultivated in a MEM medium supplemented with 10 v/v% fetal bovine serum were plated into the above dishes at an initial concentration of $2×10^4$ cells/dish, and cultivated at 37°C for 5 days in air containing 5 vol.% $CO_2$ and 7 vol.% $O_2$.

The results of cell counting in each culture dish were shown in Figure 5. In this figure, FA means a 1:1 mixture of linoleic acid and oleic acid. As a control examination, the result of cell counting obtained when the same cells were cultivated in MEM medium supplemented with 10 v/v% of fetal bovine serum was shown on the right side of the same figure.

Example 4

RITC 55-9 culture medium, whose composition is the same as that of the RITC 56-1 medium except that the former contains no mannose, galactose, lecithin or cholesterol, was added with α- or β-cyclodextrin in the amounts shown in Figure 6 and the resulting solution was sterilized by filtering through a membrane filter.

In order to examine the cytotoxicity of cyclodextrins, UMCL-3 cells were inoculated at an initial cell density of $5×10^5$/ml into RITC 55-9 medium containing various amounts of α- and β-cyclodextrin as shown in Figure 6, and cultivated at 37°C for 4 days in air containing 5% $CO_2$.

The resulting cell concentrations in the culture media were measured and are shown against the cyclodextrin concentrations in the same figure. In this figure the closed circles and the closed squares indicate the data obtained in connection with α- and β-cyclodextrin, respectively.

It is evident from the figure that β-cyclodextrin is more toxic than α-cyclodextrin and is more toxic at higher concentrations.

## Claims

1. A culture medium for mammalian cell cultures, comprising known nutrients, additives and serum or serum albumin, characterized in that the serum or serum albumin component is wholly or partially replaced by cyclodextrin and at least one lipophilic substance as a nutrient selected from unsaturated or saturated fatty acids and their glycerides, and lipophilic vitamins.

2. A culture medium according to claim 1, in which said lipophilic substance nutrient and said cyclodextrin are in the form of an inclusion complex of both components.

3. A culture medium according to claim 1 or 2, characterized in that it contains no serum or serum albumin, wherein the minimum concentration of the lipophilic substance is 0.1 mg/l in the case of fatty acids and 0.5 mg/l in the case of lipophilic vitamins, and the molar ratio of cyclodextrin to lipophilic substance is in the range of 3:1 to 500:1.

4. A culture medium according to any of claims 1 to 3, in which said cyclodextrin is α-cyclodextrin and said lipophilic substance nutrient is linoleic acid, oleic acid and/or vitamin E.

5. The use of a mixture of or of an inclusion complex between cyclodextrin and at least one lipophilic substance selected from unsaturated or saturated fatty acids and their glycerides and lipophilic vitamins useful as a cell culture nutrient as a substitute for serum or serum albumin in a culture medium for mammalian cells.

6. The use of a culture medium according to any of the claims 1 to 4 for the production of interferons, lymphokines and antibodies by cultivating mammalian cells.

7. The use according to claim 6, in which interferon is produced.

## Patentansprüche

1. Kulturmedium für die Kultur von Säugetierzellen, das bekannte Nährstoffe, Zusätze und Serum oder Serumalbumin umfaßt, dadurch gekennzeichnet, daß die Serum- oder Serumalbumin-Komponente ganz oder teilweise durch Cyclodextrin und mindestens eine lipophile Substanz, die unter ungesättigten oder gesättigten Fettsäuren und deren Glyceriden, und lipophilen Vitaminen ausgewählt wird, als Nährstoff ersetzt ist.

2. Kulturmedium gemäß Anspruch 1, in dem die als Nährstoff vorliegende lipophile Substanz und das Cyclodextrin in Form eines Einschlußkomplexes beider Bestandteile vorliegen.

3. Kulturmedium gemäß Anspruch 1 oder 2, dadurch gekennzeichnet, daß es kein Serum oder Serumalbumin enthält, wobei die Mindestkonzentration der lipophilen Substanz 0,1 g/l im Fall von Fettsäuren und 0,5 mg/l im Fall von lipophilen Vitaminen ist, und das Molverhältnis von Cyclodextrin zu lipophiler Substanz im Bereich von 3:1 bis 500:1 liegt.

4. Kulturmedium gemäß einem der Ansprüche 1 bis 3, in dem das Cyclodextrin α-Cyclodextrin ist und die als Nährstoff vorhandene lipophile Substanz Linolsäure, Ölsäure und/oder Vitamin E ist.

5. Verwendung eines Gemisches aus oder eines Einschlußkomplexes zwischen Cyclodextrin und mindestens einer lipophilen Substanz, die unter ungesättigten oder gesättigten Fettsäuren und deren Glyceriden und lipophilen Vitaminen, die als Zellkultur-Nährstoff geeignet sind, ausgewählt ist, als Ersatz für Serum oder Serumalbumin in einem Kulturmedium für Säugetierzellen.

6. Verwendung eines Kulturmediums gemäß einem der Ansprüche 1 bis 4 zur Herstellung von Interferonen, Lymphokinen und Antikörpern durch Züchten von Säugetierzellen.

7. Verwendung gemäß Anspruch 6, bei der Interferon hergestellt wird.

## Revendications

1. Un milieu de culture pour les cultures de cellules de mammifères comprenant des substances nutritives et des additifs connus et du sérum ou de la sérum-albumine, caractérisé en ce que le sérum ou la sérum-albumine sont totalement ou partiellement remplacés par une cyclodextrine et au moins une substance lipophile comme substance nutritive choisie parmi des acides gras insaturés et saturés et leurs glycérides et les vitamines lipophiles.

2. Un milieu de culture selon la revendication 1, dans lequel ladite substance nutritive lipophile et ladite cyclodextrine sont sous forme d'un complexe d'inclusion des deux composants.

3. Un milieu de culture selon la revendication 1 ou 2, caractérisé en ce qu'il ne contient pas de sérum ou de sérum-albumine, où la concentration minimale de la substance lipophile est de 0,1 mg/l dans le cas des acides gras et de 0,5 mg/l dans le cas des vitamines lipophiles et le rapport molaire de la cyclodextrine à la substance lipophile est dans la gamme de 3/1 à 500/1.

4. Un mileau de culture selon l'une quelconque des revendications 1 à 3, dans lequel ladite cyclodextrine est l'α-cyclodextrine et ladite substance nutritive lipophile est l'acide linoléique, l'acide oléique et/ou la vitamine E.

5. L'emploi d'un mélange, ou d'un complexe d'inclusion, d'une cyclodextrine et d'au moins une substance lipophile, choisie parmi les acides gras insaturés ou saturés et leurs glycérides et les vitamines lipophiles, utile comme substance nutritive pour culture cellulaire, comme produit de remplacement du sérum ou de la sérum-albumine dans un milieu de culture pour cellules de mammifères.

6. L'emploi d'un milieu de culture selon l'une quelconque des revendications 1 à 4 pour la production d'interférons, de lymphokines et d'anticorps par culture de cellules de mammifères.

7. L'emploi selon la revendication 6, dans lequel de l'interféron est produit.

FIG. 1

1

FIG. 2

FIG. 3

FIG. 4

4

FIG. 5

FIG. 6